# EUROPEAN PATENT APPLICATION

(11) **EP 3 351 210 A1**
(43) Date of publication of application: **25.07.2018**
(21) Application number: 18275008.3
(22) Date of filing: 18.01.2018
(51) Int. Cl.: A61F 2/07, A61F 2/852

(54) **BRANCH STENT RETENTION CUFF**

(30) Priority: 18.01.2017 US 201762447691 P
(71) Applicant: Cook Medical Technologies LLC, Bloomington, IN 47404 (US)
(72) Inventor: ELLER, Derek R, Huntingdon, CA 90255 (US)
(74) Representative: Williams Powell

(57) **Abstract**

The present embodiments describe an endograft (100) having at least one fenestration (170), at least one cuff support structure (210), and at least one branch stent 350), and methods for deploying the same. In one example, the system comprises a branch stent (350) within the cuff support structure (210), where upon expansion of the branch stent (350), the cuff support structure (210) expands from a first state to a second state and has a radially inward bias towards the smaller first state. In another example, the system comprises a cuff support structure (210) having at least one barb (280), where the barb is flush with the cuff support structure in a first state, and where upon expansion of the cuff support structure to a second state, the barb deflects radially inward. Both the radially inward bias of the cuff and radially inward deflection of the barb may facilitate an interference fit between the cuff and the branch stent.

## Description

### BACKGROUND

The present embodiments relate generally to medical devices, and more particularly, to endografts used to treat a diseased vessel or region of vessels.

The functional vessels of human and animal bodies, such as blood vessels and ducts, occasionally weaken or even rupture. For example, the aortic wall can weaken, resulting in an aneurysm. Upon further exposure to hemodynamic forces, such an aneurysm can rupture. One study found that in Western European and Australian men who are between 60 and 75 years of age, aortic aneurysms greater than 29 mm in diameter are found in 6.9% of the population, and those greater than 40 mm are present in 1.8% of the population.

One surgical intervention for weakened, aneurysmal, or ruptured vessels involves the use of an endoluminal prosthesis such as a stent-graft or endograft. Such a prosthesis may provide some or all of the functionality of the original, healthy vessel and/or preserve any remaining vascular integrity by replacing a length of the existing vessel wall that spans the site of vessel failure. A properly placed prosthesis excludes the diseased and/or aneurysmal portion of the vessel. For weakened or aneurysmal vessels, even a small leak ("endoleak") in or around the prosthesis may lead to the pressurization of or flow in the treated vessel which may aggravate the condition that the prosthesis was intended to treat. A prosthesis of this type can treat, for example, aneurysms of the aortic arch, thoracic aorta, abdominal aortic, iliac, or renal arteries.

In cases of aortic pathologies such as dissection or aneurysm, it is often necessary to introduce an endograft to replace or exclude the affected portion of the anatomy. Although open repair to replace a portion of the vessel may be preferable in some cases, many patients are ineligible for open surgery due to secondary issues, and require the placement of an endograft for treatment. Currently, it may be difficult to repair the aortic root through an endovascular approach, leading to poor outcomes for aortic pathologies in some patient populations.

When an aneurysm affects a main vessel, it is important to maintain flow to the peripheral vessels. The left and right coronary arteries are peripheral vessels of the aorta.

If these peripheral vessels are blocked by the main vessel prosthesis, then blood circulation is impeded, and the patient can suffer. If, for example, a coronary artery is blocked by the main vessel prosthesis, the patient can experience cardiac arrest, shortness of breath, chest pain, and reduction in blood circulation. The blockage of any peripheral vessel is usually associated with unpleasant or even life-threatening symptoms.

In general, when it is necessary to employ a connection stent between a graft and an outside vessel, the stent may be deployed with the distal end within the branch vessel, with the stent body passing through a fenestration in the graft, and some proximal portion protruding into the lumen of the graft.

The present invention seeks to provide an improved endograft and method of assembling an endograft.

### SUMMARY

According to an aspect of the present invention, there is provided an endograft for placement in a vessel of a patient as specified in claim 1.

According to another aspect of the present invention, there is provided an endograft for placement in a vessel of a patient as specified in claim 14.

In one example, the endograft may have a tubular main body having a proximal end with a proximal opening, a distal end with a distal opening, and a lumen extending therebetween. The main body may have at least one fenestration through a wall of the tubular main body portion that is in fluid communication with the main body lumen. The main body may also have at least one cuff having a proximal cuff opening, a distal cuff opening, and a cuff lumen extending therebetween. The proximal cuff opening may be attached to the at least one fenestration such that the cuff lumen is in fluid communication with the main body lumen.

Additional features may be included. For example, the cuff may have a first diameter in a first state and a second diameter in a second state, where the second cuff diameter is greater than the first cuff diameter. In the expanded second state, the cuff or a portion thereof may have a radially inward bias towards the smaller first diameter.

The cuff may also feature at least one barb. The barb may be flush with the cuff body in the first state, and in the second state the barb may deflect radially inward.

The endograft may further comprise a branch stent within at least a portion of the cuff lumen. The branch stent may have a proximal end with a proximal opening, a distal end with a distal opening, and a lumen extending therebetween. In a delivery state, the branch stent may have a first diameter and a radially outward bias towards a larger diameter. In an expanded state, the branch stent may have a second diameter greater than the first diameter and a radially outward bias towards a larger diameter. In one example, the cuff may be in the expanded state, the branch stent may be in the expanded state, and the barb may contact an outer surface of the branch stent. Furthermore, the cuff may form an interference fit with the branch stent.

The methods and systems disclosed herein are non-limiting and may be applied to other vasculature or anatomy. Other systems, methods, features, aspects and advantages of the teachings herein will be, or will become, apparent to one with skill in the art upon examination of the following figures and detailed description. It is intended that all such additional systems, methods, features and advantages be within the scope of the invention, and be encompassed by the following claims.

### BRIEF DESCRIPTION OF THE DRAWINGS

Embodiments of the present invention are described below, by way of example only, with reference to the accompanying drawings, in which the components in the Figures are not necessarily to scale, emphasis instead being placed upon illustrating the principles of the invention, and in which:
FIG. 1 is an anatomical view of the aortic root, the aortic arch, and peripheral vessels;
FIG. 2 is a side view of an embodiment of an endograft having a cuff in a relaxed state attached at a fenestration, where the cuff is cylindrical in shape;
FIG. 3 is a side view of an embodiment of an endograft having a cuff in a relaxed state, and a branch stent in a delivery state extending through the cuff lumen;
FIG. 4 is a side view of an embodiment of an endograft having a cuff in an expanded state, and a branch stent in an expanded state extending through the cuff lumen;
FIGS. 5-6 are a perspective view and an unfurled view, respectively, of an embodiment of a cuff;
FIGS. 7-8 are a perspective view and an unfurled view, respectively, of an embodiment of a cuff;
FIGS. 9-10 are a perspective view and an unfurled view, respectively, of an embodiment of a cuff;
FIGS. 11(a)-11(b) are views of a barb section of an embodiment of a cuff in a relaxed state and an expanded state, respectively;
FIG. 12 is a side view of an embodiment of an endograft having a cuff in a relaxed state attached at a fenestration, where the cuff is conical in shape; and
FIGS. 13(a)-13(b) are cross-sectional views of an embodiment of a cuff in a relaxed state and an expanded state, respectively.

### DETAILED DESCRIPTION OF THE PREFERRED EMBODIMENTS

In the present application, the term "proximal" refers to a direction that is generally upstream to the direction of blood flow during a medical procedure, while the term "distal" refers to a direction that is generally downstream to the direction of blood flow during a medical procedure.

The embodiments described below are in connection with systems and methods for the introduction and deployment of an implantable medical device in a vessel, such as endovascular prostheses, but could also be used for deploying a range of implantable medical devices including, but not limited to, stents, occlusion devices and the like.

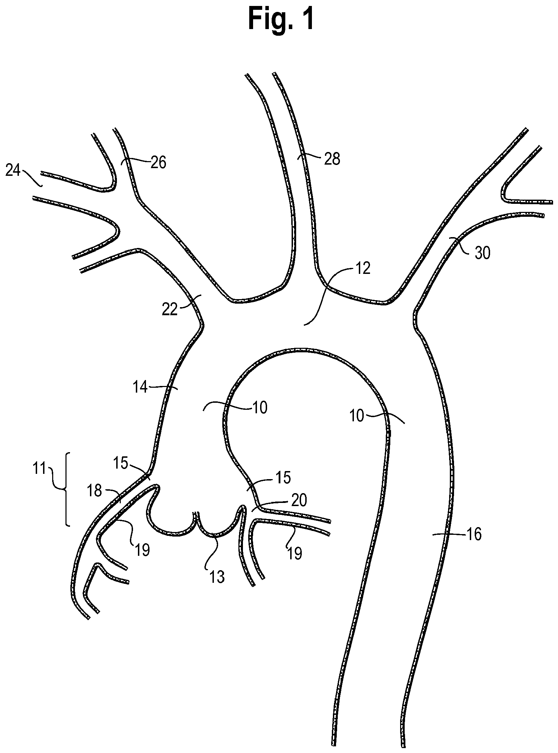

Referring to FIG. 1, the aorta 10 is the largest artery in the human body and carries blood away from the heart. FIG. 1 illustrates an example of an aortic arch 12, located distal to the ascending aorta 14 and proximal to the descending aorta 16. The aortic root 11 is the section of the aorta 10 closest to the heart, and includes the aortic valve 13 and coronary ostia 15. The right coronary artery 18 and left coronary artery 20 are peripheral vessels 19 in the ascending aorta 14 and circulate blood to the heart tissue itself. Other peripheral vessels 19 near the aortic arch 12 include the brachiocephalic artery 22, right subclavian artery 24, right common carotid artery 26, left common carotid artery 28, and left subclavian artery 30.

Over time, the walls of the aorta 10 and other vessels may lose elasticity or otherwise weaken. Due to hemodynamic pressure, the vessel walls of the aorta 10 may expand in diameter, resulting in an aneurysm. While an aneurysm by itself is not an acute problem, it can increase the risk of a possibly fatal vessel rupture if the aneurysm expands and/or bursts. A common treatment for the aneurysm is to relieve the pressure on the aneurysm by redirecting blood flow through a stent graft or endograft.

Endografts may be implanted in the aorta 10, such that blood flows through the endograft, avoiding the aneurysm. Use of an endograft reduces pressure on the aneurysm and can cause the aneurysm to shrink in size. Endografts may incorporate self-expanding stents. The shape, size, and position of the endograft may also be modified through use of a balloon catheter. Aortic endovascular repair may be complicated in cases such as aortic root dilation (or dissection which originates in the aortic root or ascending aorta) by the fact that a prosthesis deployed there may block perfusion to the coronary arteries.

To provide blood flow to these arteries and peripheral vessels 19, endografts may have one or more openings called fenestrations. Covered branch stents (or branch endografts) may extend through one or more fenestrations to cannulate these arteries. The branch stent may be deployed with the distal end within the peripheral vessel 19, the branch stent body passing through a fenestration in the graft, and some proximal portion of the branch stent protruding into the lumen of the endograft. This fenestration method may be effective, but may have disadvantages or be unusable in certain applications. For example, in the case of an endograft which is intended for use in the ascending aorta, which would be intended to exclude at least a portion of the aortic sinus, an endograft may require branch stents to maintain blood flow to the coronary arteries. A branch stent extending through the fenestration and having a proximal portion protruding into the lumen of the endograft could interfere with the leaflets of the aortic valve, potentially causing damage to the valve or the stent. In addition, the dramatic motion and high pressures experienced in that area, a single ring of contact to hold the branch stent may not be sufficient to maintain a hemodynamic seal and to prevent mobility of the branch stent.

In some embodiments , covered branches may be attached to an endograft prior to deployment. Attached branches may be within the lumen of the endograft or outside of the graft main body, and may be made from a similar material to the graft itself and supported by stents or other support structure similar to the support structure of the endograft. However, attached covered branches may increase the delivery profile of the device. Additionally, the presence of internal branches within the endograft lumen may lead to unfavorable turbulence in flow through the endograft.

An endograft having a cuff attached to a fenestration and configured to operatively attach to a branch stent may allow the branch stent to maintain flow to a peripheral artery without protruding into the endograft lumen, thus avoiding interference with the aortic valve leaflets, avoiding increased turbulent flow through the endograft, and avoiding an increased delivery profile of the endograft.

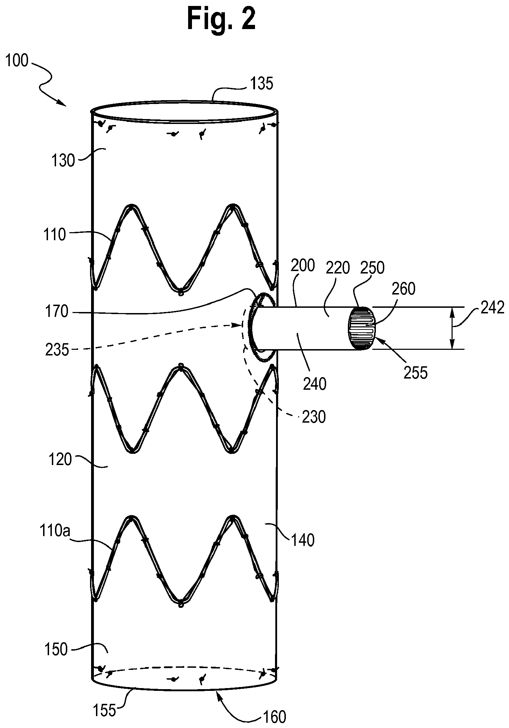

FIGS. 2-4 illustrate an embodiment of an endograft 100 in a pre-cannulated state, a cannulated relaxed state, and a cannulated expanded state, respectively. Endograft 100 may comprise an expandable support structure 110 and a graft material 120, including a tubular main body 140 having a proximal end 130 with a proximal opening 135, a distal end 150 with a distal opening 155, and a lumen 160 extending therebetween. The proximal opening 135 and distal opening 155 may both provide fluid access to the lumen 160 of the main body 140. The main body 140 may be generally tubular in shape, and have either a uniform or varying diameter along its length. The main body 140 may include at least one fenestration 170 through a wall of the tubular main body 140, where the at least one fenestration 170 is in fluid communication with the lumen 160.

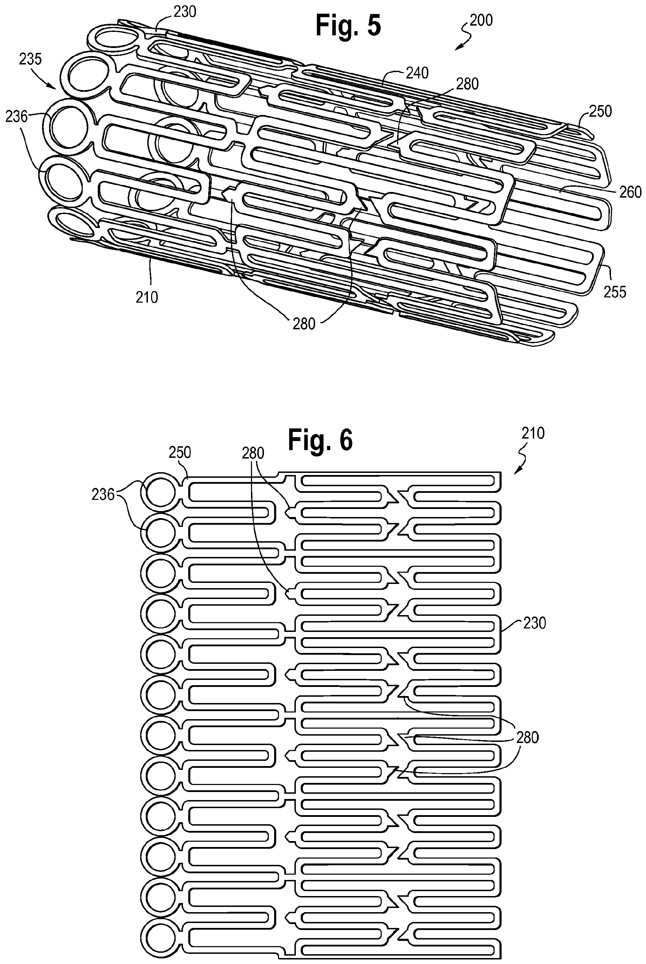

The main body 140 may include at least one cuff 200 comprising a cuff body 240 having a proximal end 230 with a proximal cuff opening 235, a distal end 250 with a distal cuff opening 255, and a cuff lumen 260 extending therebetween. The proximal cuff opening 235 may be attached to at least one fenestration 170 such that the cuff lumen 260 is in fluid communication with the main body lumen 160. The cuff body 240 may be in a relaxed state having a first (reduced) cross-sectional diameter 242. The cuff body 240 may expand and/or contract, depending on the presence or absence of forces applied to the cuff 200. The cuff body 240 may also comprise a cuff support structure 210, a cuff covering 220, and at least one barb(s) 280 (shown in FIGS. 5-11). Although only one cuff 200 is illustrated here, endograft 100 may include one or more cuffs 200 to accommodate the appropriate anatomy of one or more peripheral vessels 19. For example, an embodiment designed for the right and left coronary arteries 18 and 20, respectively, may have two cuffs 200, one for each artery. Similarly, an endograft 100 designed to access the renal arteries (not shown) may also have two cuffs 200. Other potential target anatomy include the brachiocephalic artery, left carotid artery, and left subclavian arteries in the aortic arch, as well as the celiac and superior mesenteric in the abdominal aorta. Any vessel requiring the addition of branch stents may be target vessel.

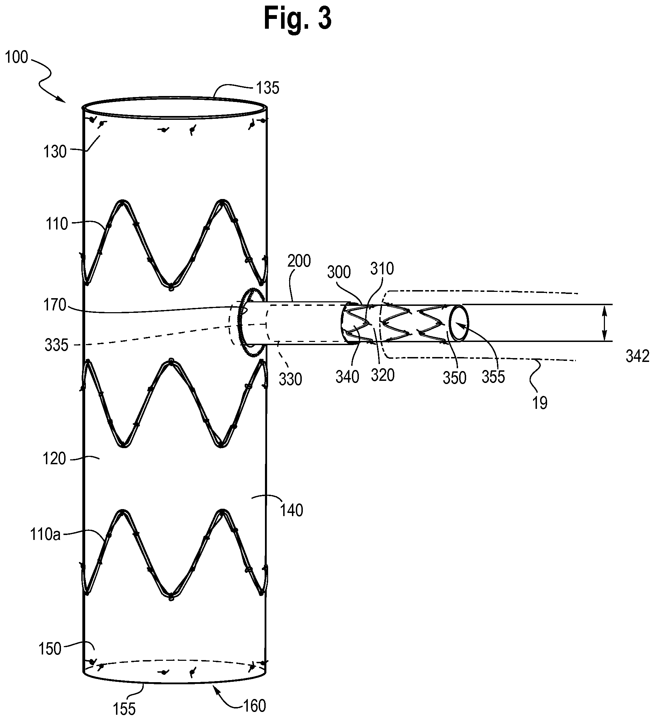

As shown in FIG. 3, a branch stent 300 may cannulate the lumen 260 of cuff 200 and extend through to cannulate a peripheral vessel 19, for example, right coronary artery 18 and/or left coronary artery 20. The branch stent 300 may comprise an expandable support structure 310 (e.g., laser-cut balloon expandable covered stents, laser-cut self-expandable covered stents, laser-cut balloon expandable segmented covered stents, external Z-stents, or internal Z-stents) and a biocompatible graft material 320, including a tubular branch stent body 340 having a proximal end 330 with a proximal opening 335, a distal end 350 with a distal opening 355, and a branch stent lumen 360 extending therebetween. The branch stent lumen 360 may be in fluid communication with the main body lumen 160 and the cuff lumen 260. The branch stent body 340 may expand and contract, depending on outside forces or the lack thereof.

As shown in FIG. 3, the cuff body 240 may be in a relaxed state having a first (reduced) cross-sectional diameter 242, and the branch stent body 340 may be in a delivery state having a first (reduced) cross-sectional diameter 342.

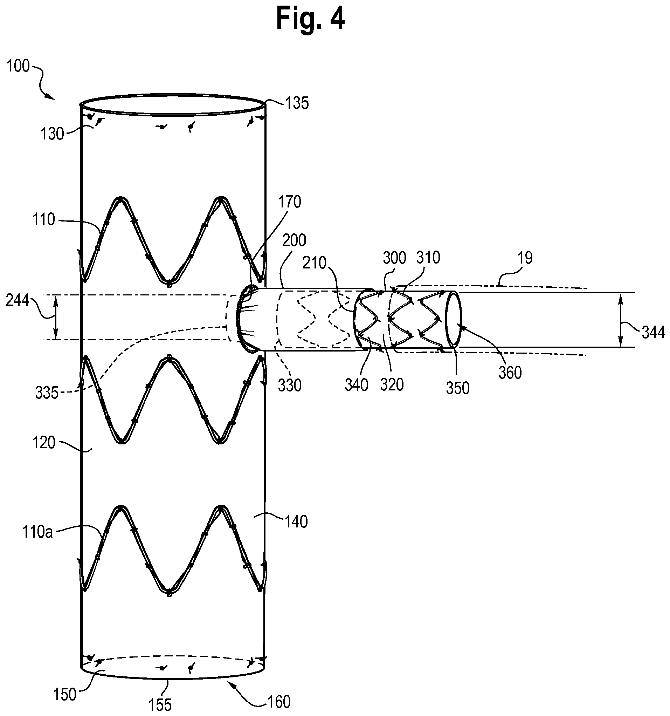

As shown in FIG. 4, cuff body 240 may expand to an expanded state having a second (increased) cross-sectional diameter 244 greater than the first cross-sectional diameter 242, and may have a radially inward bias. The radially inward bias may originate from one or both of the cuff support structure 210 or the cuff covering 220. The branch stent body 340 may also expand to an expanded state having a second (increased) cross-sectional diameter 344 greater than the first cross-sectional diameter 342.

As shown in FIGS. 2-4, the support structure 110 of the endograft 100 may have any suitable stent pattern known in the art. The support structure 110 may be self-expanding or may expand under external pressures, for example from an inflatable balloon at the tip of a balloon catheter. One example of a stent pattern is the Z-stent or Gianturco stent design. Each Z-stent may include a series of substantially straight segments or struts interconnected by a series of bent segments or bends. The bent segments may include acute bends or apices. The Z-stents are arranged in a zigzag configuration in which the straight segments are set at angles relative to one another and are connected by the bent segments. Alternative stents may include, for example, annular or helical stents. The stents mentioned herein may be made from standard medical grade stainless steel. Other stents may be made from nitinol or other shape-memory materials. Similarly, branch stent support structure 310 may comprise any of the embodiments of support structure 110.

As shown in FIGS. 2-4, the main body 140 of endograft 100 and branch stent body 340 of branch stent 300 may each comprise at least one support structure 110 (or 310), such as a stent. The support structure 110 (or 310) may include a single, unitary structure or a plurality of independent structures. The support structure 110 (or 310) and/or various portions thereof may be disposed on the inner surface and/or outer surface of the graft material 120 (or 320). Multiple support structures 110 (or 310) may be positioned at any point or points along a length of endograft 100 (or branch stent 300), as generally depicted in FIGS. 2-4.

In the current, non-limiting example, a plurality of external Z-stents 110a are disposed external to the graft material 120 at spaced-apart locations along the endograft 100. Internal Z-stents 110b may also be disposed along portions of the main body 140. Given varying design configurations, external Z-stents 110a may be replaced with internal Z-stents 110b, and vice versa.

The graft material 120 (or 320) may be connected to the one or more support structures 110 (or 310) by known methods, for example biocompatible stitching. The graft material 120 (or 320) may be fabricated from any at least substantially biocompatible material including such materials as polyester fabrics, polytetrafluoroethylene (PTFE), expanded PTFE, and other synthetic materials known to those of skill in the art. In some embodiments in accordance with the technology, the graft material 120 (or 320) may also include drug-eluting coatings or implants. Cuff covering 220 may also comprise any material appropriate for the graft materials 110 or 310, and/or may also comprise a thin-walled elastomeric material such as silicone or polyurethane. Alternatively, cuff 200 may have no covering.

The tubular main body 140 may be contiguous with the proximal end 130 including the proximal opening 135, and the distal end 150 including the distal opening 155. The lumen 160 may extend within the interior of the main body 140, extending between the proximal opening 135 and distal opening 155. The proximal opening 135 and distal opening 155 may both provide fluid access to the lumen 160 of the main body 140. The main body 140 may be generally tubular in shape, and have either a uniform or varying diameter along its length.

When deploying the endograft 100 into a region with at least one peripheral vessel 19 to be cannulated, such as the aorta 10 in the region of aortic root 11 near the right and left coronary arteries 18 and 20, respectively, it may be desirable to provide at least one fenestration 170 through a wall of the tubular main body 140, where the fenestration 170 is in fluid communication with the lumen 160. Alternatively, the fenestration(s) 170 may be located through a wall of the proximal end 130 or distal end 150. For illustrative purposes herein, embodiments with only one fenestration will be described. Each of these embodiments, their equivalents, and other embodiments understood by one skilled in the art, may also have additional fenestrations 170.

The fenestration 170 may be circular, though other appropriate shapes may be utilized. The fenestration 170 may be reinforced along the perimeter to provide structural support, for example using internal or external Z-stents (not shown) or biocompatible stitching. The fenestration 170 may be configured to attach to the proximal opening 235 of the proximal end 230 of the cuff 200, and may be sized for cannulation by branch stent 300 where the branch stent 300 is in the delivery state. As with all embodiments, radiopaque or MRI opaque markers may be used to define the periphery of the fenestration 170. In alternative embodiments targeting other anatomy, other numbers of fenestrations 170 may also be used.

The cuff 200 may comprise the cuff covering 220 wrapped around a cuff support structure 210 (shown in detail in FIGS. 5-11). It may be attached to the cuff support structure 210 via biocompatible stitching, forming the cuff body 240. The cuff body 240 may be contiguous with the proximal cuff end 230, including the proximal cuff opening 235, and the distal cuff end 250, including the distal cuff opening 255. The cuff lumen 260 may extend within the interior of the cuff body 240, extending between the proximal cuff opening 235 and distal cuff opening 255. The proximal cuff opening 235 and distal cuff opening 255 may both provide fluid access to the cuff lumen 260, and thereby fluid access to the endograft lumen 160.

The cuff 200 may attach to the endograft 100 by attaching the proximal cuff opening 235 to the fenestration 170 and/or the graft material 120 at or near the fenestration 170. Alternatively, the cuff 200 may attach internally to the endograft 100 by attaching to the surface of the endograft lumen 160. Any suitable attachment mechanism may be utilized, for example, biocompatible stitching. As shown in FIGS. 5-6, the proximal cuff end 230 may comprise suture holes 236 through which biocompatible stitches may be sewn to the graft material 120 at or near the fenestration 170. To accommodate this connection, the proximal opening 235 may have a substantially similar shape to fenestration 170, for example, a circular cross-section. Similarly, the distal opening 255 may have a substantially similar shape to the proximal end 330 of branch stent 300, for example, a circular cross-section.

The cuff body 240 may be made from nitinol ("NiTi"), or a similar shape-memory type alloy. The cuff body 240 may be manufactured with struts in tortious, folded arrangements, which may expand radially. Alternatively, it could be made from silicone, polyurethane, or a similar elastomeric material, and contain embedded metal features, possibly similar to the stents mentioned above, either within the cuff body 240 (attached internally or externally), to allow for expansion and increased strength and radial force.

The branch stent 300 may comprise the graft material 320 wrapped around the expandable support structure 310. The expandable support structure may be made from any appropriate material, for example, materials appropriate for the expandable support structure 110, as described above. Similarly, graft material 320 may be made from any material appropriate, for example, materials appropriate for graft material 120, as described above. Graft material 320 may be attached to the expandable support structure 310 via biocompatible stitching, forming the branch stent body 340. The branch stent body 340 may be contiguous with the branch stent proximal end 330 including the proximal opening 335, and the distal end 350 including the distal opening 355. The branch stent lumen 360 may extend within the interior of the branch stent body 340, extending between the proximal opening 335 and distal opening 355. The branch stent proximal opening 335 and distal opening 355 may both provide fluid access to the branch stent lumen 360. The branch stent body 340 may be generally tubular in shape, and have either a uniform or varying diameter along its length.

In use, endograft 100 may be deployed intravascularly, for example in the aortic root 11 or ascending aorta 14. The delivery system containing the graft may be tracked from a distal approach (e.g., transfemoral) and guided over the aortic arch 12. Because rotational alignment is important, the system may optionally utilize a pre-curved cannula core to rotationally orient the system relative to the aortic arch 12, for example, embodiments of the system of U.S. Pat. No. 8,394,135. The main body 140 of the endograft 100 may be deployed by retraction of an outer sheath (not shown). The endograft 100 may employ diameter reduction ties (not shown) to prevent full deployment at this stage. The target peripheral vessel 19, for example the right coronary artery 18 or left coronary artery 20, may be cannulated with a catheter using standard wire and catheter techniques and passing through the endograft lumen 160, fenestration 170 and cuff lumen 260. After cannulation, if diameter reduction ties are in place, they may be removed to fully deploy the graft. Branch stent 300 may be tracked over the standard wire cannulating the target peripheral vessel 19, passing through the endograft lumen 160, fenestration 170, cuff lumen 260, and into the target peripheral vessel 19. As shown in FIG. 3, the distal end 350 of the branch stent 300 may enter the peripheral vessel 19, while the proximal end 330 of the branch stent 300 remains within the cuff lumen 260. After deployment, wires and catheters may be removed from the system. In some embodiments, a transcatheter heart valve (not shown) may be deployed into the proximal end 130 of the endograft 100, for example at the proximal opening 135.

After the branch stent 300 is within the peripheral artery 19, the expandable support structure 310 of branch stent 300 may expand, for example, via expansion of a balloon-tipped catheter for expandable branch stents 300, or removal of a sheath for self-expanding branch stents 300. As support structure 310 increases in diameter, the branch stent body 340 may also increase in diameter. Eventually, the branch stent body 340 may contact the cuff lumen 260 and may begin to apply radially outward pressure on the cuff body 240 (since the branch stent 300 is cannulated within cuff lumen 260). As a result of the radially outward forces applied to the cuff body 240 by the expansion of the branch stent body 340, the cuff body 240 may also increase in diameter to an expanded state.

In a relaxed state (FIGS. 2-3), the cuff body 240 may have a first diameter 242. In an expanded state (FIG. 4), the cuff body 240 may have a second diameter 244 greater than the first diameter 242. In the expanded state, the cuff body 240 may have a radially inward bias from the larger second diameter 244 towards the smaller first diameter 242, which inward bias may originate from one or both of the cuff support structure 210 or the cuff covering 220.

Unlike the cuff 200, in a compressed state (FIGS. 2-3), the branch stent body 340 may have a first diameter 342. In an expanded state (FIG. 4), the branch stent body 340 may have a second diameter 344 greater than the first diameter 342. In both the compressed state and the expanded state, the branch stent body 340 may have a radially outward bias towards a diameter greater than the second diameter 344. Thus, in the expanded state (FIG. 4), the outward bias of the branch stent body 340 may oppose the inward bias of the cuff body 240, creating an interference fit between the proximal end 330 of branch stent body 340 and the cuff lumen 260. This interference fit may sealingly engage the branch stent body 340 and the cuff lumen 260, forming a hemodynamic seal. In some embodiments, for balloon-expandable branch stents 300, the branch stent 300 may have no significant radial bias in the expanded state, yet the radially inward bias of the cuff 200 may nonetheless form an interference fit and hemodynamic seal with the branch stent 300.

The proximal end 330 of the branch stent body 340 may engage the cuff lumen 260 anywhere along the axial length of the cuff 200, including along the full length of the cuff lumen 260. If the proximal end 330 of the branch stent body 340 engages the cuff lumen 260 along less than the entire axial length of the cuff lumen 260, the cuff covering 220 may cover the non-overlapping portion to prevent blood from escaping via the openings in the cuff support structure 210. Thus, the cuff graft material 220 may preferably cover the entire axial length of the cuff body 240 so as to prevent leakages regardless of the axial location of branch stent 300.

Upon expansion, the distal end 350 of branch stent body 340 may sealingly engage the inner surface of peripheral vessel 19. Thus, the main body 140, cuff body 240, and branch stent body 340 may form a telescoping connection with the peripheral vessel 19. In this expanded state, the lumen of peripheral vessel 19 remains in fluid communication with the aorta 10, the main body lumen 160, the cuff lumen 260, and the branch stent lumen 360.

The radially inward bias of the cuff 200 may be caused by the materials from which the cuff body 240 is manufactured, as described above, and may originate from one or both of the cuff support structure 210 or the cuff covering 220. For example, cuff 200, including cuff support structure 210, may be manufactured from a shape-memory type alloy designed to return to a relaxed state having the first cuff diameter 242. Thus, when appropriate forces are applied, the cuff body 240 may expand to an expanded state having an increased diameter 244, but the shape-memory alloy properties may bias the cuff body 240 back towards the relaxed state having first diameter 242, forming an interference fit with the surface(s) applying the radially outward force(s). Further, if the radially outward force(s) are reduced or removed, the shape-memory alloy properties may bias the cuff body 240 back towards the relaxed state having first diameter 242. This inward force biasing the cuff body 240 towards the first diameter 242 may be in equilibrium with any radially outward force of the expandable support structure 310 of branch stent 300. For example, if the branch stent expandable support structure 310 is balloon-expandable to an expanded state, the cuff 200 may only compress the branch stent 300 minimally, if at all, before reaching equilibrium, though the radially inward force from the cuff 200 to the branch stent 300 may be enough to form the interference fit. This interference fit may sealingly engage the branch stent body 340 and the cuff lumen 260, forming a hemodynamic seal. The inward bias of the cuff body 240 may cause the cuff body 240 to closely conform to the outer surface of the branch stent body 340, decreasing the chance of leakage between the cuff 200 and branch stent 300. This may also provide greater securement of the branch stent 300 within the cuff 200 due to the continued radial forces. This securement may be further improved by the use of barbs 280, as discussed below.

FIGS. 5-6 illustrate a detailed perspective view and unfurled view, respectively, of an embodiment of cuff 200 without cuff covering 220. Cuff 200 comprises the cuff support structure 210 wrapped by cuff covering 220 (not shown) to form the cuff body 240. The cuff body 240 may be contiguous with the proximal cuff end 230, including the proximal cuff opening 235, and the distal cuff end 250, including the distal cuff opening 255. The cuff lumen 260 may extend within the interior of the cuff body 240, extending between the proximal cuff opening 235 and distal cuff opening 255. The proximal cuff opening 235 and distal cuff opening 255 may both provide fluid access to the cuff lumen 260. The proximal cuff opening 235 may further comprise at least one suture hole 236 and at least one barb 280.

As shown in FIGS. 5-6, a plurality of suture holes 236 may be disposed along the perimeter of the proximal cuff opening 235. Biocompatible stitches may be sewn through the suture holes 236 and into the graft material 120 at or near the fenestration 170, attaching the cuff support structure 210 (and hence the cuff 200) to the endograft 100. To accommodate this connection, the proximal opening 235 may have a substantially similar shape to fenestration 170, for example, a circular cross-section. When sewn to the graft material 120 of endograft 100, the suture holes 236 may flex outward so as to conform to the outer surface of the endograft body 140 near fenestration 170. An inward-flex would not be preferred since it may obstruct blood flow. Thus, the embodiment illustrated in FIG. 5 is pre-attachment, indicated by the absence of flexing at the suture holes 236.

As shown in FIGS. 5-6, a plurality of barbs 280 may be disposed around cuff support structure 210. The barbs 280 may be oriented longitudinally (as illustrated in FIGS. 5-6), latitudinally, radially, obliquely, or at other angles. Cuff 200 may have any number of barbs 280. The embodiment shown in FIG. 5 illustrates the cuff 200 in a relaxed state. The barb(s) 280 may be flush with the cuff main body 240 in the relaxed state, and in the expanded state the barb(s) 280 may deflect radially inward. This radially-inward deflection of at least one barb 280 may further facilitate the connection between cuff 200 and branch stent 300 to form a hemodynamic seal. This is because the barb(s) 280 may interact with the exterior surface of the branch stent body 340, including the expandable support structure 310. Portions of the expandable support structure 340 may catch on the barb(s) 280, securing an interference fit. This may be in addition to, or independent of, the interference fit caused by the radially inward bias of the cuff 200, described above. Alternatively, the barb(s) 280 may even facilitate a hemodynamic seal between the cuff 200 and branch stent 300 without deflecting inward, for example, if the radially outward expansion of the branch stent 300causes the expandable support structure 310 to become enmeshed, ensnared, entangled, or otherwise catch on barb(s) 280. This interaction between the barb(s) 280 and the expandable support structure 310 may be significant, causing a large degree of interference and securing the two together during and after expansion.

The barb pattern of the cuff 200 may be manufactured from any suitable method. For example, a NiTi cuff may be laser cut to form the unfurled barb pattern of FIG. 6. The laser-cut metal sheet may then be formed into a cylindrical shape and attached (e.g., ultrasonic welding), so as to form a one-piece cuff 200.

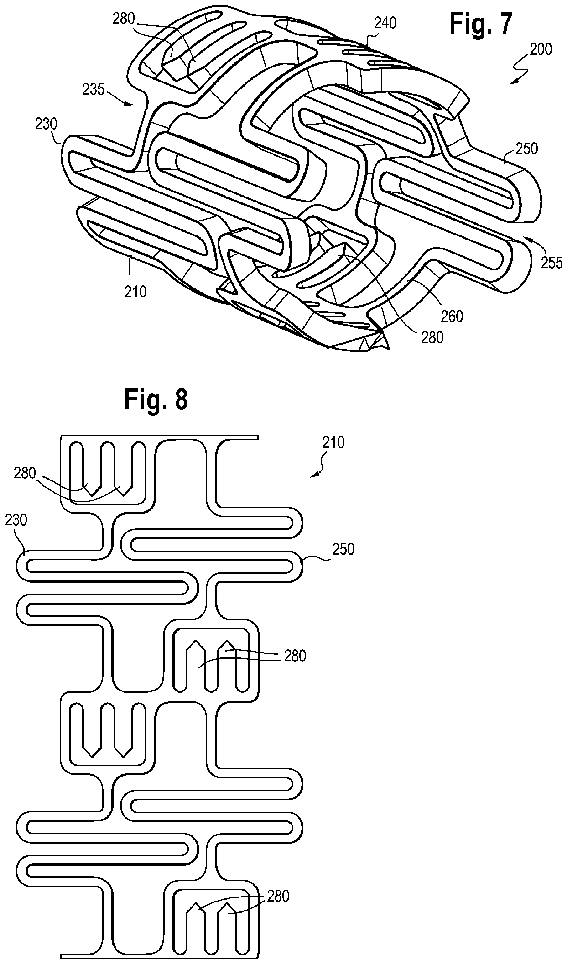

FIGS. 7-8 illustrate a detailed perspective view and unfurled view, respectively, of an embodiment of cuff 200, including cuff support structure 210, without cuff covering 220. This embodiment does not include suture holes 236, which may be optionally added. At least one barb 280 may be oriented latitudinally, for example as shown in FIGS. 7-8. The embodiment shown in FIG. 7 illustrates the cuff 200 in a relaxed state. The barb(s) 280 may be flush with the cuff main body 240 in the relaxed state, and in the expanded state the barb(s) 280 may deflect radially inward. This radially-inward deflection of at least one barb 280 may further facilitate the connection between cuff 200 and branch stent 300 to form a hemodynamic seal, similar to the embodiment illustrated in FIGS. 5-6.

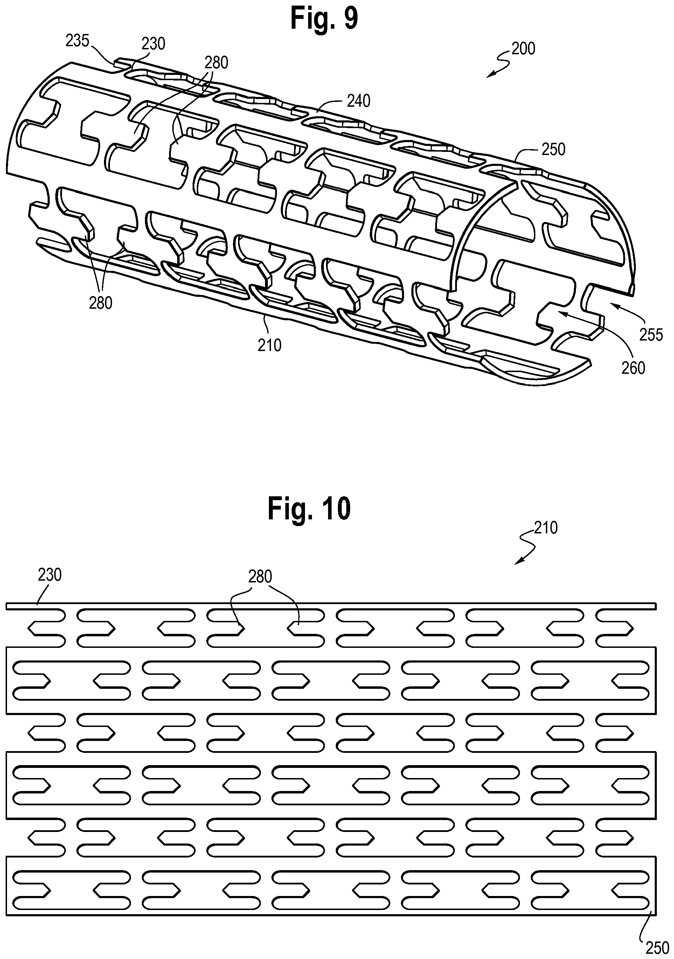

FIGS. 9-10 illustrate a detailed perspective view and unfurled view, respectively, of an embodiment of cuff 200, including cuff support structure 210, without cuff covering 220. This embodiment does not include suture holes 236, which may be optionally added. At least one barb 280 may be oriented latitudinally, for example, as shown in FIGS. 9-10. The embodiment shown in FIG. 9 illustrates the cuff 200 in a relaxed state. The barb(s) 280 may be flush with the cuff main body 240 in the relaxed state, and in the expanded state the barb(s) 280 may deflect radially inward. This radially-inward deflection of at least one barb 280 may further facilitate the connection between cuff 200 and branch stent 300 to form a hemodynamic seal, similar to the embodiments illustrated in FIGS. 5-8.

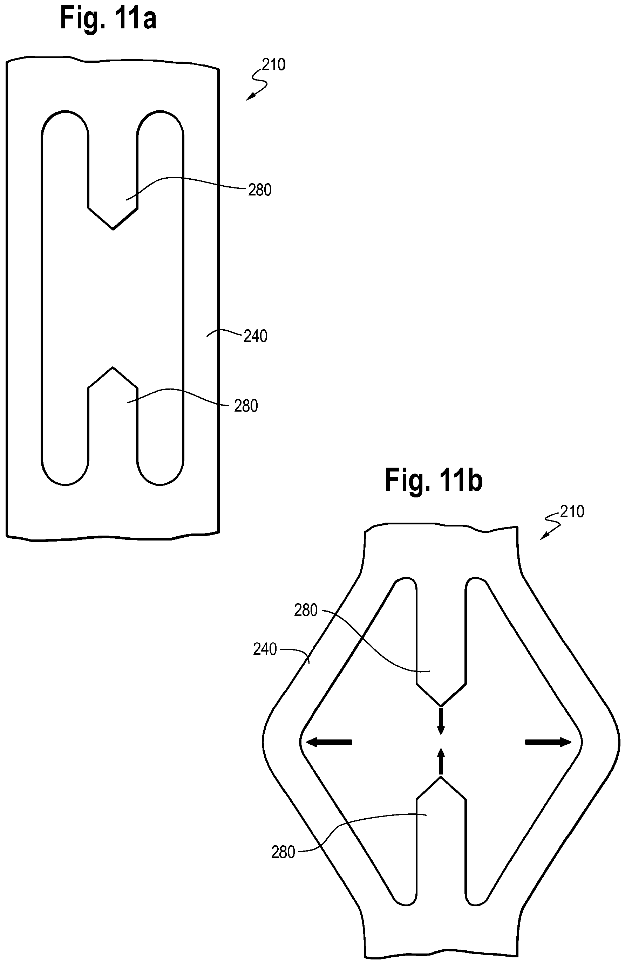

FIGS. 11(a)-(b) illustrate a zoomed view of a barb 280 of cuff support structure 210 in the relaxed state and expanded state, respectively. In the relaxed state shown in FIG. 11(a), the barb(s) 280 may be flush with the cuff body 240. In the expanded state shown in FIG. 11(b), the cuff body 240 expands to a greater diameter, while the barb(s) 280 may deflect radially inward into the lumen space of the cuff 200 and/or barbs 280 may by pulled towards one another, increasing contact and imparting forces on any portion of the expandable support structure 310 (or any graft material) between barbs 280. In some embodiments, there may be provided a single barb 280 within the space formed by the cuff body 240 struts rather than two opposing barbs 280 as shown in Figures 11a and 11b. In a similar manner to the two opposing barb embodiment, the arrangement with a single barb will trap any portion of the expandable support structure 310 (or any graft material) from a side branch when the cuff support structure 210 changes from its first relaxed state to its expanded state, specifically between the barb and the cuff supporting structure. This will occur as a result of the fact that the barb tip will move radially inwardly relative to the cuff support structure 210 when the cuff expands from the relaxed state to the expanded state.

In another example, the cuff support structure 210 may have at least one barb 280 having a barb tip, the barb tip being at a first distance to a facing part of the cuff support structure 210 when the cuff support structure 210 is in a relaxed state having a first cuff diameter. The cuff support structure 210 may expand to a second diameter greater than the first diameter. Upon expansion, the barb 28 may be at a second distance to said facing part of the cuff support structure 210, where said second distance may be less than the first distance.

In another example, the cuff support structure 210 may have first and second opposing barbs 280 with facing barb tips, the barbs tip being at a first distance to one another when the cuff support structure is in a relaxed state having a first cuff diameter. The cuff support structure 210 may expand to a second diameter greater than the first diameter. Upon expansion, the opposing barb tips may be at a second distance to one, said second distance being less than the first distance.

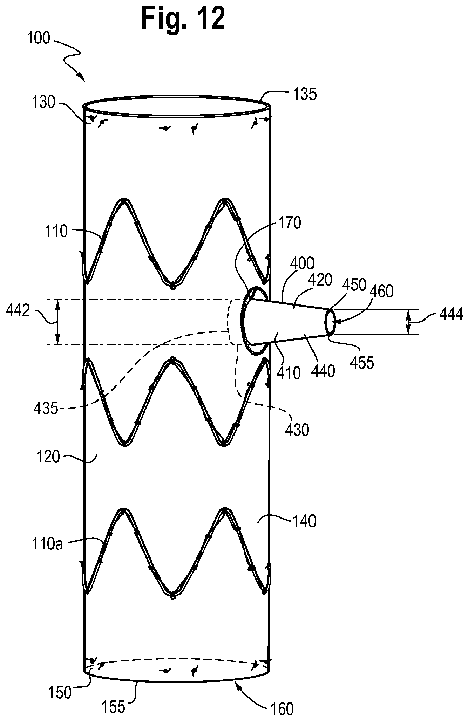

Another embodiment is illustrated in FIG. 12. This embodiment is similar to the embodiments of FIGS. 2-4, however, the embodiment of FIG. 12 discloses cuff 400. The main body 140 may include at least one cuff 400 comprising a cuff body 440 having a proximal end 430 with a proximal cuff opening 435, a distal end 450 with a distal cuff opening 455, and a cuff lumen 460 extending therebetween. The proximal cuff opening 435 may be attached to at least one fenestration 170 such that the cuff lumen 460 is in fluid communication with the main body lumen 160.

The cuff body 440 may be in a relaxed state having a tapered cross-sectional diameter, tapering from a first cross-sectional diameter 442 at the proximal end 430 to a second cross-sectional diameter 444 at the distal end 450. The first cross-sectional diameter 442 may be greater than the second cross-sectional diameter 444, creating the tapered effect.

The cuff body 440 may expand and/or contract, depending on the presence or absence of forces applied to the cuff 400. The cuff body 440 may also comprise a support structure 410, a cuff covering 420, and at least one barb 480 (for example, as illustrated in FIGS. 5-11). Although only one cuff 400 is illustrated here, endograft 100 may include one or more cuffs 400 to accommodate the appropriate anatomy of one or more peripheral vessels 19, as described above.

Similar to the embodiments shown in FIGS. 3-4, a branch stent 300 may be cannulated within cuff 200 and a peripheral vessel 19. Upon expansion of the branch stent 300, the cuff 400 may expand from a relaxed state to a radially expanded state as a result of the radially outward forces from the branch stent 300 on the cuff 400. In the radially expanded state, the cross-sectional diameters along the axial length of the cuff 400 may be greater than or equal to the cross-sectional diameters in the relaxed state. In other words, the cross-sectional diameter at the proximal end 430 may be greater than or equal to the first diameter 442, and the cross-sectional diameter at the distal end 450 may be greater than or equal to the second diameter 444. Thus, in the expanded state, the cuff 400 may or may not maintain the tapered shape, depending on the degree of diameter changes along the axial length of the cuff 400. For example, cuff 400 may be substantially cylindrical in the expanded state if it expands to the same shape as a cylindrical branch stent 300.

In the expanded state, cuff 400 may have a radially inward bias towards the relaxed state having a tapered cross-sectional diameter along an axial length (from a first cross-sectional diameter 442 at the proximal end 430 to a second cross-sectional diameter 444 at the distal end 450). Similar to the embodiments of FIGS. 2-4, this radially inward bias may originate from one or both of the cuff support structure 410 and the cuff covering 420, and may help to facilitate an interference fit between the branch stent 300 and cuff 400, forming a hemodynamic seal. The bias of expanded cuff 400 towards the tapered relaxed state may further facilitate this connection because, in order for the branch stent 300 to slide distally (in the direction of blood flow), the branch stent 300 must slide through ever-smaller cross-sectional space, further increasing the interference fit.

The cuff 400 may further comprise any of the embodiments illustrated in FIGS. 5-11 having at least one barb 480, wherein the cuff body 440 may be tapered as described above. The barb(s) 480 may be flush with the cuff main body 440 in the relaxed state, and in the expanded state the barb(s) 480 may deflect radially inward. This radially-inward deflection of at least one barb 480 may further facilitate the connection between cuff 400 and branch stent 300 to form a hemodynamic seal, similar to the embodiments illustrated in FIGS. 5-11.

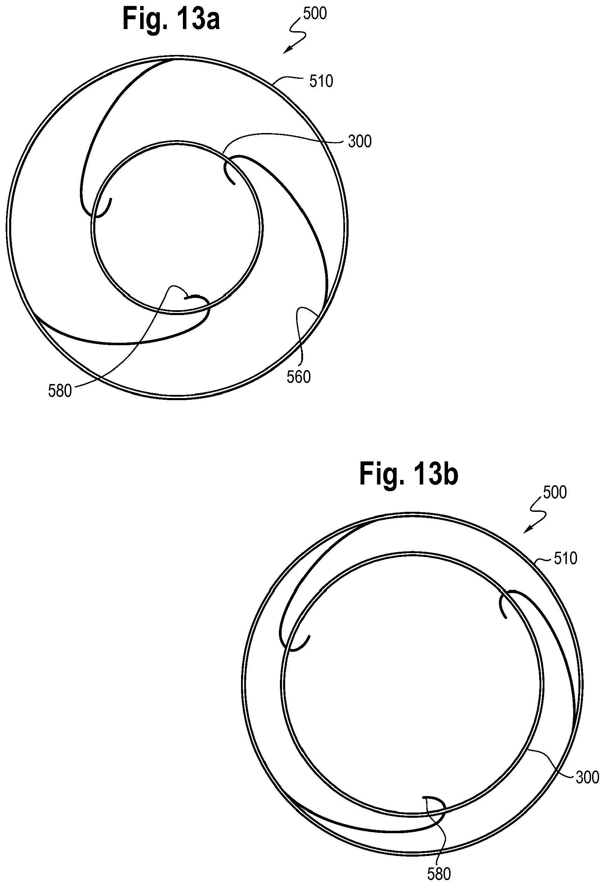

FIGS. 13(a)-(b) illustrate cross-sectional views (along longitudinal axis) of an alternative embodiment of an endograft cuff 500 in relaxed and expanded states, respectively. As shown, the branch stent 300 is cannulated and coaxial within cuff 500. Cuff 500 may have a cuff support structure 510, a lumen 560, and one or more hook(s) 580 extending into the lumen 560. For illustrative purposes, three hooks 580 are shown in FIGS. 13(a)-(b), although any number of hooks 580 may be utilized. Hooks 580 may be disposed radially around the cuff 500 so as to extend into the lumen 560. Hooks 580 may be radially anchored to some degree, but may be capable of expanding, for example, as the branch stent 300 and cuff 500 expand. The hooks 580 may loop around or otherwise interface with branch stent 300, for example, latching on to expandable support structure 310 (shown in other figures). The hooks 580 may be rotationally stable since they are affixed to cuff 500. As a result, the distance between them increases as the branch stent 300 expands into the cuff, placing an increased strain on the branch stent 300 in the expanded state (e.g., pulling on the expandable support structure 310). This may serve as an additional connection mechanism between the cuff 500 and branch stent 300.

In the embodiment illustrated in FIG. 13, the hooks 580 are depicted as individual units extending into the lumen 560 of cuff 500. Alternatively, the hooks 580 may sit primarily flush with the cuff body 540 (shown in other figures) with only the barbs of the hooks 580 protruding into the cuff lumen 560.

In other examples, the cuff 200 (or 400) could be made as a flexible, deformable sleeve, for example, from an elastomeric material. The radially inward bias of the cuff 200 (or 400) could originate simply based on the elastic recoil of the elastomeric material. The elastic sleeve may contain metallic or other hard elements such as barbs, but have a structure that is primarily based on the elastic sleeve.

In another embodiment, the cuff 200 (or 400) may comprise a self-expanding stent which has been heat-set to a smaller diameter than the second (greater) cross-sectional diameter 344 of branch stent 300. The self-expanding stent may be covered with a suitable graft material 120, or may be uncovered. The self-expanding stent may be secured to the fenestration 170, for example via biocompatible stitching. The branch stent 300 may then be cannulated through the self-expanding stent, and expanded as described above. Since the self-expanding stent has been heat-set, it will resist expansion beyond the heat-set limit, thereby providing the radially inward bias described in other cuff embodiments. In some embodiments, this section of stent may have one or more of its struts heat-set to be pointing radially inward towards the lumen of the stent to form a barb.

The embodiments described herein may provide an endograft with a reduced delivery profile. This is because the branch stent 300 may be delivered separately from the endograft 100. Furthermore, the required length and width of cuff 200 may be reduced due to the barb(s) 280 and radially inward bias which secure the branch stent 300 to the cuff 200, reducing the surface area required to create the interference fit. In embodiments with no cuff covering 220, the delivery profile may be further reduced.

The embodiments described herein may provide certain benefits since the branch stent 300 may be fully deployed within the cuff lumen 260, as opposed to having the proximal end 330 extending within the endograft lumen 160. This may reduce turbulent blood flow through the endograft lumen 160 and the aorta 10 or other surrounding vessels. Additionally, in target anatomy such as the ascending aorta 14 where the branch stent 300 may be deployed within a coronary artery 18 or 20, deploying the branch stent 300 fully within the cuff lumen 260 may avoid interference with the aortic valve leaflets 13. The leaflets may extend distally towards the endograft 100 during systole when the aortic valve opens and blood is ejected from the ventricle, and contact with the endograft 100 or other structures such as branch stent 300 may not be desirable. Furthermore, given the relatively dramatic motions of the ventricular and ascending aortic anatomy, the radially inward bias of the cuff 200 combined with barb(s) 280 may both act to reduce the likelihood of the branch stent 300 slipping or otherwise becoming unsecured within the cuff lumen 260. An externally mounted cuff 200 may further reduce turbulent flow and interference.

The embodiments described herein provide non-limiting examples of endografts that are suitable for treating an array of medical conditions, and may be especially suited for treating an aortic aneurysm at or slightly above the aortic root 11. Various additional modular components may be provided for the endograft 100, for example, additional cuffs 200 and branch stents 300 may be utilized.

While references to treatment of an aortic aneurysm at or near the aortic root 11 may be explained as one example, it will be appreciated that endografts 100 and 200 can be positioned at other bodily locations to treat aneurysms or other conditions, using the system and methods described herein.

While various embodiments of the invention have been described, the invention is not to be restricted except in light of the attached claims and their equivalents. Moreover, the advantages described herein are not necessarily the only advantages of the invention and it is not necessarily expected that every embodiment of the invention will achieve all of the advantages described.

The disclosures in United States patent application no. 62/447,691 filed on January 18, 2017, from which this application claims priority, and in the abstract accompanying this application are hereby incorporated herein in their entirety by reference.

## Claims

1. An endograft for placement in a vessel of a patient, comprising:
a tubular main body portion having a proximal end with a proximal opening, a distal end with a distal opening, and a lumen extending therebetween;
at least one fenestration through a wall of the tubular main body portion that is in fluid communication with the main body lumen;
at least one cuff comprising a cuff support structure and having a proximal cuff opening, a distal cuff opening, and a cuff lumen extending therebetween,
where the proximal cuff opening is attached to the main body at the fenestration such that the cuff lumen is in fluid communication with the main body lumen,
where in a first state, the cuff support structure has a first diameter,
where in a second state, the cuff support structure has a second diameter greater than the first diameter, and has a radially inward bias towards the smaller first diameter, and
wherein the cuff support structure has at least one barb, wherein the barb is flush with the cuff support structure in the first state, and the barb is positioned radially inwardly of the support structure in the second state.

2. An endograft according to claim 1, comprising a branch stent having a proximal end with a proximal opening, a distal end with a distal opening, and a lumen extending therebetween, wherein a portion of the branch stent is within the cuff lumen.

3. An endograft according to claim 2, where in a delivery state, the branch stent has a first diameter and a radially outward bias towards a larger diameter, and where in an expanded state, the branch stent has a second diameter greater than the first diameter and a radially outward bias towards a larger diameter.

4. An endograft according to claim 3, wherein the cuff support structure is in the expanded state, the branch stent is in the expanded state, and the at least one barb contacts the branch stent.

5. An endograft according to claim 2, 3 or 4, wherein the cuff support structure and the branch stent are connected via an interference fit.

6. An endograft according to any preceding claim, comprising a covering attached to an outer surface of the cuff support structure.

7. An endograft according to any preceding claim, wherein the barb extends substantially parallel to a longitudinal axis of the cuff support structure.

8. An endograft according to any preceding claim, wherein the barb extends substantially perpendicular to a longitudinal axis of the cuff support structure.

9. An endograft according to any preceding claim, wherein the barb comprises at least two protrusions extending in opposite directions.

10. An endograft according to any preceding claim, wherein the proximal cuff opening comprises suture holes.

11. An endograft according to any preceding claim, wherein the cuff support structure has a cylindrical shape.

12. An endograft according to any preceding claim, wherein the cuff support structure has a conical shape.

13. An endograft for placement in a vessel of a patient, comprising:
a tubular main body portion having a proximal end with a proximal opening, a distal end with a distal opening, and a lumen extending therebetween;
at least one fenestration through a wall of the tubular main body portion and in fluid communication with the main body lumen;
at least one cuff comprising a cuff support structure and having a proximal cuff opening, a distal cuff opening, and a cuff lumen extending therebetween,
where the proximal cuff opening is attached to the at least one fenestration such that the cuff lumen is in fluid communication with the main body lumen, and
where the cuff support structure has at least one barb having a barb tip, the barb tip being at a first distance to a facing part of the cuff support structure when the cuff support structure has a first cuff diameter, and being at a second distance to said facing part of the cuff support structure when the cuff support structure has a second cuff diameter,
said second cuff diameter being greater than the first cuff diameter, and
said second distance being less than the first distance.

14. An endograft according to claim 13, wherein the cuff support structure has first and second opposing barbs with facing barb tips, the barb tips being at a first distance to one another when the cuff support structure has a first cuff diameter, and being at a second distance to one another when the cuff support structure has a second, greater, cuff diameter, said second distance being less than the first distance.

15. An endograft according to claim 13 or 14, wherein the at least one barb extends substantially parallel to a longitudinal axis of the cuff support structure.
